# EUROPEAN PATENT SPECIFICATION

(11) **EP 4 287 863 B1**
(45) Date of publication and mention of the grant of the patent: **26.11.2025**
(21) Application number: 22703363.6
(22) Date of filing: 02.02.2022
(51) Int. Cl.: A24F 40/20, A24F 40/46, A61M 11/04, A61M 15/06, A61M 15/00

(54) **NON-COMBUSTIBLE AEROSOL PROVISION DEVICE**
VORRICHTUNG ZUR BEREITSTELLUNG EINES AEROSOLS OHNE VERBRENNUNG
DISPOSITIF DE FOURNITURE D'AÉROSOL SANS COMBUSTION

(30) Priority: 03.02.2021 GB 202101468
(43) Date of publication of application: 13.12.2023
(60) Divisional application: 25209869.4
(73) Proprietor: Nicoventures Trading Limited, London WC2R 3LA (GB)
(72) Inventor: CAMPBELL, Jeremy, London WC2R 3LA (GB); WARREN, Luke James, London WC2R 3LA (GB); BIJL, Erik-Jouke, 3991 (NL); WRIGHT, Matthew, Cambridge Cambridgeshire CB1 1HZ (GB); WALTON, Daniel Anthony, Cambridge Cambridgeshire CB1 1HZ (GB)
(74) Representative: Dehns
(86) International application number: PCT/EP2022/052460
(87) International publication number: WO 2022/167472

(56) References cited:
- WO-A1-2020/025746
- US-A1- 2016 360 794
- US-A1- 2020 054 074
- US-A1- 2020 060 340

## Description

### Technical Field

The present invention relates to a non-combustible aerosol provision device and a non-combustible aerosol provision system comprising a non-combustible aerosol provision device and a consumable comprising aerosol-generating material from which the non-combustible aerosol provision device is configured to generate an aerosol.

### Background

Smoking articles such as cigarettes, cigars and the like burn tobacco during use to create tobacco smoke. Attempts have been made to provide alternatives to these articles that burn tobacco by creating products that release compounds without burning. Examples of such products are heating devices which release compounds by heating, but not burning, the material. The material may be for example tobacco or other non-tobacco products, which may or may not contain nicotine.

US 2020/054074 A1 discloses an adjustable vaporizer that receives atomizers having different dimensions. **In** one embodiment, an adjustable vaporizer is provided that includes an adjustable aperture located at a top portion of the adjustable vaporizer that adjusts to receive atomizers having a range of diameters. The adjustable vaporizer also includes an adjustable atomizer chamber aligned with the adjustable aperture that adjusts to receive atomizers having a range of lengths.

WO 2020/025746 A1 discloses a system comprising an aerosol-generating device. The aerosol-generating device comprises a heating chamber. The heating chamber is configured to receive an aerosol-generating article of a first type. The aerosol-generating article comprises aerosol-forming substrate. The system further comprises an adapter element for insertion into the heating chamber of the aerosol-generating device. The adapter element is configured having an outer shape to be at least partly inserted into the heating chamber of the aerosol-generating device. The adapter element has a cavity extending along the longitudinal axis of the adapter element. The cavity is configured so that an aerosol- generating article of a second type having a diameter which is smaller than the diameter of the aerosol-generating article of the first type can be inserted into the cavity and thereby into the heating chamber.

US 2020/060340 A1 discloses an aerosol delivery device. In various implementations, the aerosol delivery device comprises a control body having an outer housing, an electrical energy source located within the housing, a control component operatively connected to the electrical energy source, a heating assembly operatively connected to the control component, and an aerosol source member that includes an aerosol generating component configured to be positioned proximate the heating assembly. The heating assembly comprises a series of heating members, and each heating member is independent and distinct and configured to heat a segment of the aerosol source member.

US 2016/360794 A1 discloses a heating device for a cigarette including a housing, a heating chamber, and a power supply. The heating chamber is arranged at a first end of the housing, and is configured for receiving at least one part of the cigarette. The heating chamber defines an air inlet and an opening. The opening is configured for receiving an end of the cigarette. The heating chamber includes a plurality of heating pieces arranged on two opposite inner walls. Each heating piece includes a heating surface facing an interior space of the heating chamber. The power supply is arranged at an opposite second end of the housing, and is configured for feeding the heating pieces power.

### Summary

The invention is defined in claim 1 wherein the dependent claims define advantageous embodiments thereof.

Further features and advantages of the invention will become apparent from the following description of preferred embodiments of the invention, given by way of example only, which is made with reference to the accompanying drawings.

### Brief Description of the Drawings

Figure 1 shows a simplified schematic representation of an example of a non-combustible aerosol provision device according to the invention;
Figure 2 shows a side view of another example non-combustible aerosol provision device according to the invention;
Figure 3 shows a cross-sectional side view of the non-combustible aerosol provision device of Figure 2;
Figure 4A and Figure 4B show cross-sectional side views of the non-combustible aerosol provision device of Figures 2 and 3, showing, respectively, a first consumable and a second consumable being received by the device;
Figure 5A shows a perspective view of an example heating chamber for an example non-combustible aerosol provision device;
Figure 5B shows a perspective cross-sectional view of the heating chamber shown in Figure 5A in combination with an isolation tube;
Figure 6 shows, in a perspective cross-sectional view, another example of a non-combustible aerosol provision device according to the invention;
Figure 7A and Figure 7B show in a perspective cross-sectional view a heating chamber of the device of Figure 6 in receipt of, respectively, a first consumable and a second consumable;
Figure 7C and Figure 7D show, respectively, schematic top-down views of the arrangements shown in Figure 7A and 7B;
Figure 8 shows, in a perspective cross-sectional view, another example of a non-combustible aerosol provision device according to the invention;
Figure 9A and Figure 9B show in cross-sectional side views, a heating chamber of the device of Figure 8 in receipt of different consumables;
Figure 9C and Figure 9D show close-up cutaway views of, respectively, portions of the arrangements shown in Figure 9A and Figure 9B;
Figure 10A shows a side view of an example of a heating tube for use with a non-combustible aerosol provision device according to the invention; and
Figure 10B and Figure 10C show close-up cross-sectional side views of a portion of the heating tube of Figure 10A when the heating tube is in receipt of different consumables.

### Detailed Description

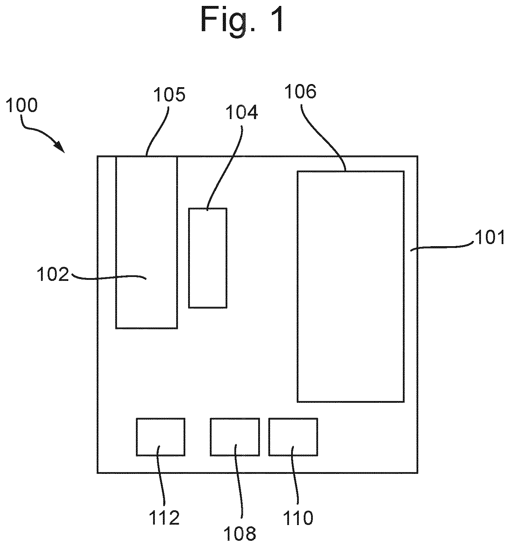

Figure 1 is a simplified schematic view of an example non-combustible aerosol provision device 100. The non-combustible aerosol provision device 100 comprises a heating chamber 102. The heating chamber 102 is configured to receive a consumable (not shown in Figure 1) which comprises aerosol-generating material, which may or may not comprise tobacco.

Aerosol-generating material is a material that is capable of generating aerosol, for example when heated, irradiated or energized in any other way. Aerosol-generating material may, for example, be in the form of a solid, liquid or gel which may or may not contain an active substance and/or flavourants. In some embodiments, the aerosol-generating material may comprise an "amorphous solid", which may alternatively be referred to as a "monolithic solid" (i.e. non-fibrous). In some embodiments, the amorphous solid may be a dried gel. The amorphous solid is a solid material that may retain some fluid, such as liquid, within it. In some embodiments, the aerosol-generating material may for example comprise from about 50wt%, 60wt% or 70wt% of amorphous solid, to about 90wt%, 95wt% or 100wt% of amorphous solid.

The aerosol-generating material may comprise one or more active substances and/or flavours, one or more aerosol-former materials, and optionally one or more other functional material.

The active substance as used herein may be a physiologically active material, which is a material intended to achieve or enhance a physiological response. The active substance may for example be selected from nutraceuticals, nootropics, psychoactives. The active substance may be naturally occurring or synthetically obtained. The active substance may comprise for example nicotine, caffeine, taurine, theine, vitamins such as B6 or B12 or C, melatonin, cannabinoids, or constituents, derivatives, or combinations thereof. The active substance may comprise one or more constituents, derivatives or extracts of tobacco, cannabis or another botanical.

In some embodiments, the active substance comprises nicotine. In some embodiments, the active substance comprises caffeine, melatonin or vitamin B 12.

The one or more other functional materials may comprise one or more of pH regulators, colouring agents, preservatives, binders, fillers, stabilizers, and/or antioxidants.

The aerosol-former material may comprise one or more constituents capable of forming an aerosol. In some embodiments, the aerosol-former material may comprise one or more of glycerol, propylene glycol, diethylene glycol, triethylene glycol, tetraethylene glycol, 1,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate.

A consumable is an article comprising or consisting of aerosol-generating material, part or all of which is intended to be consumed during use by a user. A consumable may comprise one or more other components, such as an aerosol-generating material storage area, an aerosol-generating material transfer component, an aerosol generation area, a housing, a wrapper, a mouthpiece, a filter and/or an aerosol-modifying agent. A consumable may also comprise an aerosol generator, such as a heater, that emits heat to cause the aerosol-generating material to generate aerosol in use. The heater may, for example, comprise combustible material, a material heatable by electrical conduction, or a susceptor.

In this example, the non-combustible aerosol provision device 100 is for heating aerosol-generating material in a consumable to volatilise at least one component of the aerosol-generating material. The non-combustible aerosol provision device 100 is hereafter referred to as the device 100. The device 100 is configured to heat the aerosol-generating material in a consumable comprising aerosol-generating material (not shown in Figure 1) which is received in the described heating chamber 102. The device 100 comprises a heating arrangement 104 configured to provide energy for heating the aerosol-generating material in a consumable received in the heating chamber 102. In some examples, the heating arrangement 104 comprises one or more resistive heating elements arranged in thermal contact with the heating chamber 102. The flow of current against the electrical resistance of the one or more resistive heating elements generates heat. This process is called Joule, ohmic, or resistive heating.

A susceptor is a material that is heatable by penetration with a varying magnetic field, such as an alternating magnetic field. The susceptor may be an electrically-conductive material, so that penetration thereof with a varying magnetic field causes induction heating of the heating material. The heating material may be magnetic material, so that penetration thereof with a varying magnetic field causes magnetic hysteresis heating of the heating material. The susceptor may be both electrically-conductive and magnetic, so that the susceptor is heatable by both heating mechanisms. The device that is configured to generate the varying magnetic field may also be referred to as a magnetic field generator.

In some examples, the heating arrangement 104 is an induction heating arrangement and is configured to generate a varying magnetic field in order to inductively heat a susceptor. In some examples, the susceptor is configured to define the heating chamber 102 for receipt of the consumable, as will be described below in more detail. The induction heating arrangement may comprise one or more inductors through which an alternating current is passed to generate the varying magnetic field. In some examples using induction heating, the heating arrangement 104 comprises one or more susceptors. In other examples using induction heating, the heating arrangement 104 may not comprise a susceptor and one or more susceptors may instead be provided as part of/with consumables intended for use with the device 100.

The device 100 comprises a power source 106. The power source 106 supplies electrical power to the various components of the device 100. In some examples, the power source 106 is a battery. In some examples, the power source 106 comprises a battery and a DC-DC converter, and power is supplied from the battery through the DC-DC converter. The DC-DC converter may allow the power supply 106 to supply power at a different voltage to the voltage of the battery. In some examples, the device 100 may comprise a DC to AC converter for converting a DC current from, e.g., a battery to AC current, for example, to supply power to one or more inductors of the heating arrangement 104 where the heating arrangement 104 is an induction heating arrangement. In the following examples, the power source 106 is referred to simply as the battery 106.

In the example of Figure 1, the device 100 comprises a processor 108 in data communication with a computer readable memory 110. The processor 108 is configured to control various aspects of the operation of the device 100. The processor 108 controls the various aspects by executing instructions stored on the computer readable memory 110. For example, the processor 108 may control the operation of the heating arrangement 104. For example, the processor may control the delivery of electrical power from the battery 106 to the heating arrangement 104 by controlling various electrical components such as switches and the like (not shown in Figure 1).

The device 100 comprises a housing 101 which forms an outer cover of the device 100 and surrounds and houses various components of the device 100. The chamber 102 in this example is configured to receive a consumable (not shown in Figure 1) comprising aerosol-generating material from which the device 100 is configured to generate aerosol. The device 100 may generate aerosol from the consumable by the heating aerosol-generating material, for example, in the ways described above. In other examples, an aerosol may be generated from the aerosol-generating material by imparting energy to the aerosol-generating material in another way, for example, by use of ultrasonic energy.

The device 100 has an opening 105 in one end, which allows the consumable to be inserted into the chamber 102. As shown in certain examples below, in some examples a portion of the consumable may be received in the chamber 102 of the device 100, while a portion of the consumable may protrude from the opening 105 of the device 100. In examples, the portion of the consumable received in the chamber 102 comprises the aerosol-generating material from which the device 100 is configured to generate aerosol. The portion of the consumable which protrudes from the opening 105 may, for example, comprise a filter or the like, and the user may inhale the generated flow of aerosol from the portion of the consumable which protrudes from the opening 105 by inserting that portion into his or her mouth.

The device 100 may comprise a user-operable control element 112, such as a button or switch, which operates the device 100 when pressed. For example, a user may turn on the device 100 by operating the switch 112.

The device 100 may also comprise an electrical component, such as a socket/port (not shown), which can receive a cable to charge the battery 106. For example, the socket may be a charging port, such as a USB charging port. **In** some examples the socket may be used additionally or alternatively to transfer data between the device 100 and another device, such as a computing device. The socket may also be electrically coupled to the battery 106 via electrical tracks.

The end of the device 100 closest to the opening 105 may be known as the proximal end (or mouth end) of the device 100 because, in use, it is closest to the mouth of the user. **In** use, a user may insert the consumable into the opening 105, operate the user control 112 to begin heating the aerosol-generating material and draw on the proximal end of the device 100. This causes the aerosol to flow through the device 100 along a flow path towards the proximal end of the device 100. **In** examples, a portion of the consumable may protrude from the opening 105 and the user may draw on a proximal end of the consumable to cause the aerosol to flow to the proximal end of the device 100 to be inhaled. **In** other examples, the device 100 may comprise a mouthpiece on which the user inhales to draw the flow of aerosol.

An end of the device 100 opposite the proximal end and furthest away from the opening 105 may be known as the distal end of the device 100 because, in use, it is the end furthest away from the mouth of the user. As a user draws on the aerosol generated in the device 100, the aerosol flows away from the distal end of the device 100.

The device 100 may in some examples comprise a lid/cap (not shown), arranged towards the distal end of the device 100. Opening the lid/cap may provide access to the heating chamber 102. A user may, for example, open the second lid to clean components in the heating chamber 102, e.g. to remove debris from previous usage sessions.

The chamber 102 is configured to accommodate, one at a time, a first consumable and a second consumable (not shown in Figure 1). The chamber 102 is configured such that, the first consumable when received in the chamber 102 is accommodated at a first position, while the second consumable when received in the chamber 102 is accommodated at a second position different from the first position.

The first consumable and the second consumable have different dimensions to one another. For example, the first consumable and the second consumable may have different lengths and/or have different diameters. In some examples, the first consumable and the second consumable are elongate consumables, which may, for example, be substantially cylindrical. The first and second consumables both comprise aerosol-generating material. The first and second consumables may, for example, comprise a distal portion containing the aerosol-generating material. The first and second consumables may also comprise a proximal portion which may comprise, for example, a filter and/or other components. Examples of the first and second consumables are described in more detail below, with reference to later figures. It should be noted that, herein, reference to consumables of different sizes or to differently-dimensioned consumables refers to consumables which are intended to be a different size to another, and does not, for example, refer to consumables which are intended to be the same size but differ in size by some small amount, due to, e.g., manufacturing tolerances. The consumables may, for example, be of different types to one another wherein each of the different types of consumables is intended to have different dimensions.

Since the chamber 102 is configured to accommodate either of the first consumable and the second consumable, the device 100 may accordingly be used by a user with either of the first consumable or the second consumable. The chamber 102 may be such that it requires no reconfiguration to accommodate either consumable since the first consumable can be accommodated at a first position in the chamber 102 while the second consumable can be accommodated at a second position in the chamber 102.

The heating chamber 102 may, in some examples, be defined by a heating tube (not shown in Figure 1). The heating tube may surround the heating chamber 102 such that an interior hollow of the heating tube defines the heating chamber 102. The heating tube may be configured to be heated and to thereby heat a consumable received in the heating chamber 102. For example, the heating arrangement 104 may comprise one or more inductive elements and the heating tube may comprise a susceptor material and be configured to be inductively heated by the one or more inductive elements. **In** other examples, the heating tube may be configured to be heated by one or more resistive elements. For example, the heater tube may be configured to be formed of or in contact with a resistive heater, such as a thin film heater. Examples of such arrangements are discussed below in more detail. **In** other examples, the heating tube may itself not be configured to heat the consumable but may contain the consumable while the consumable is being heated. For example, the consumable may comprise susceptor material to be heated by one or more inductive elements of the heating arrangement 104 to thereby generate the flow of aerosol.

The heating tube may have a circular cross-section or may have a cross-section of another shape. The adjustment mechanism may allow a dimension of the heating chamber 102 to be adjusted by adjusting a diameter of the heating tube. For example, a diameter of the heating tube may be adjustable to adjust the diameter of the heating chamber 102. Additionally, or alternatively, a length of the heating tube may be adjustable to adjust the length of the heating chamber.

**In** certain examples, the chamber 102 of the device 100 may be configured to accommodate other consumables having different dimensions to either the first consumable or the second consumable. For example, the chamber 102 may be configured to accommodate each of a plurality of consumables at different respective positions in the chamber 102.

It will be appreciated that the device 100 may comprise other components not shown in Figure 1, such as ventilation inlets/outlets, a control interface, etc. It should be noted that Figure 1 is merely a schematic sketch showing a number of components that may be included in the device 100. Figure 1 is not intended to communicate particular positions of various components.

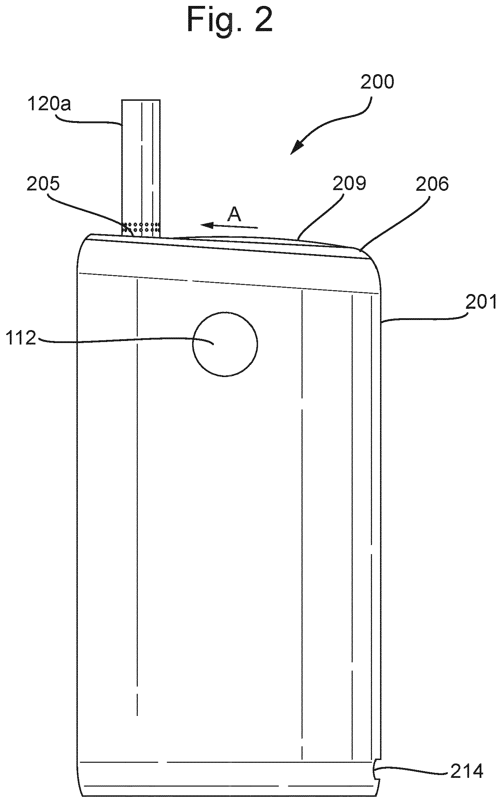

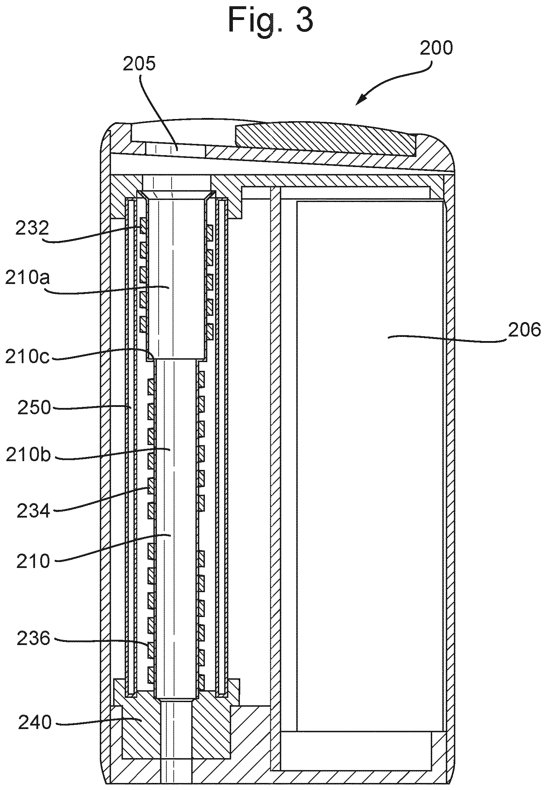

Figure 2 shows a side view of a second device 200 according to an example of the invention. Figure 3 shows a side view of the second device 200 in a schematic representation wherein the outer cover is omitted such that details of the chamber 202 and other interior components of the second device 200 are shown. The second device 200 may comprise any of the features of the device 100 which have been described above with reference to Figure 1 and discussion of these features will not be repeated here. Like figure references will be used to denote those features already described with reference to Figure 1.

In Figure 2, a first consumable 120a is shown received in the heating chamber 102 of the device 200, to be heated. A portion of the first consumable 120a protrudes from the opening 105.

The device 200 comprises a heating tube 210, shown in Figure 3. An interior of the heating tube 210 defines a heating chamber 202. The heating tube 210, in this example, is a conductive heating tube which is configured to be heated resistively by a plurality of heating coils 232, 234, 236.

In this example, the heating tube 210 comprises a first section 210a and a second section 210b. The first section 210a is located toward the proximal end of the device 200. The second section 210b is located toward the distal end of the device 200. The first section 210a and the second section 210b are located end to end axially. A distal end of the first section 210a communicates with a proximal end of the second section 210b. The first section 210a has a first diameter and the second section 210b has a second diameter which is less than the first diameter. The opening 105 allows insertion of a consumable, such as the first consumable 120a, into the first section 210. Since the diameter of the second section 210b is less than the diameter of the first section 210a, only a consumable having a diameter less than or equal to the diameter of the second section 210b may be inserted into the second section 210b. A consumable having a diameter which is too great to allow the consumable to be inserted into the second section 210b abuts a step 210c in the heating tube 210.

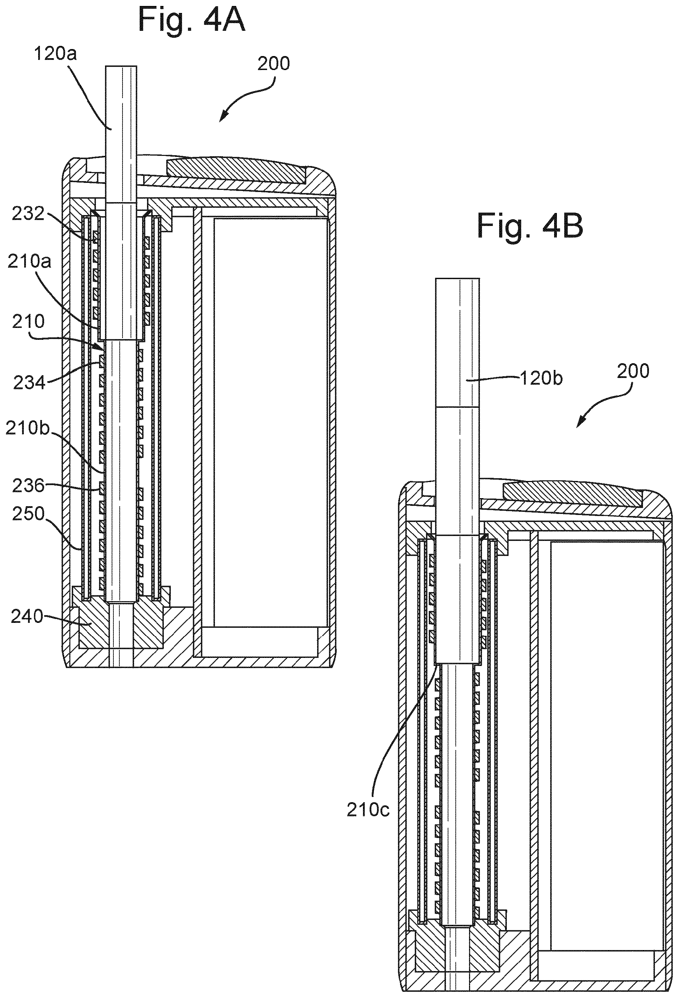

Accordingly, the difference in diameter between the first section 210a and the second section 210b allows the heating tube 210 to accommodate consumables having different dimensions at different points in the heating tube 210. An example is shown in Figures 4A and 4B.

In this example, the first consumable 120a has a diameter which is less than a diameter of a second consumable 120b, shown in Figure 4B. In one example, the first consumable 120a has a diameter of around 5.40mm while the second consumable 120b has a diameter of around 6.68mm. In some examples, the first consumable 120a and the second consumable 120b have different lengths to one another. In one example, the first consumable 120a has a length of around 83mm while the second consumable 120b has a length of around 75mm.

In Figure 4A the first consumable 120a is shown accommodated in the heating tube 210. The first consumable 120a has a diameter which allows it to be inserted into the second section 210b of the heating tube 210. The first consumable 120a is of a length such that it can be inserted all the way to a distal end of the heating tube 210 at which is located a distal stop 240. The distal stop 240 is configured to be abutted by the distal end of the first consumable 120a to position the first consumable 120a in the heating tube 210.

It can be seen from Figure 4A that since the first consumable 120a has a diameter less than that of the first section 210a, a gap is present between the first consumable 120a and the interior surface of the first section 210a. For example, an annular gap may be present around the first consumable 120a in the first section 210a. In some examples, the gap may allow the first section 210a to act as an expansion chamber for the flow of aerosol generated from the first consumable 120a which is heated in the second section 210b.

In Figure 4B the second consumable 120b is shown accommodated in the heating tube 210. The second consumable 120b has a diameter which is greater than the diameter of the second section 210b of the heating tube 210. Accordingly, the second consumable 120a can be inserted through the opening 105 into the first section 210a but cannot be inserted into the second section 210b. A distal end of the second consumable 120b accordingly abuts the step 210c between the first section 210a and the second section 210b.

The heating tube 210, in this example, is a conductive heating tube which may be formed of, for example, a metal such as steel. The heating tube 210 is configured to be heated and to conduct heat to whichever of the first consumable 120a and the second consumable 120b is received in the heating tube 210. In examples, the device 200 may be configured to heat different sections of the heating tube 210 independently, for example, by using a separate heating element for different section of the heating tube 210. In such examples, the heating elements may be controllable independently of one another. In this example, the heating tube 210 is arranged to be heated by the plurality of coils 232, 234, 236. A first heating coil 232 is arranged to heat the first section 210a of the heating tube 210. The first heating coil 232 in this example is wound around the first section 210a. A second heating element 234 and a third heating element 236 are arranged to heat respectively a proximal zone and a distal zone of the second section 210b. Accordingly, the second section 210b in this example is split into two separate heating zones.

The first, second and third coils 232, 234, 236 may be independently operated to heat a desired portion of the heating tube 210. For example, when the second consumable 120b is received in the heating tube 210 as shown in Figure 4B, the first coil 232 may be operated to heat the first section 210a of the heating tube 210 to heat the second consumable 120b. However, when the first consumable 120a is received in the heating tube 210 as shown in Figure 4A, the second coil 234 and the third coil 236 may be operated as desired to heat their respective heating zones in the second section 210b of the heating tube 210. In examples, the first coil 232 may remain inoperable while the first consumable 120a is received in the heating tube 210.

An isolation tube 250 surrounds the heating tube 210 and isolates the heating tube 210 from other components of the device 200. The isolation tube 250 may be generally tubular and at least partially surround the susceptor tube 210. The isolation tube 250 may be constructed from an insulating material, such as a plastics material for example. In this particular example, the isolation tube 250 is constructed from polyether ether ketone (PEEK). The isolation tube 250 may help insulate the various components of the device 200 from the heat generated by the coils 232, 234, 236. The isolation tube 250 may also be configured to prevent the egress of materials from the chamber 202 into portions of the device 200 exterior to the isolation tube 250.

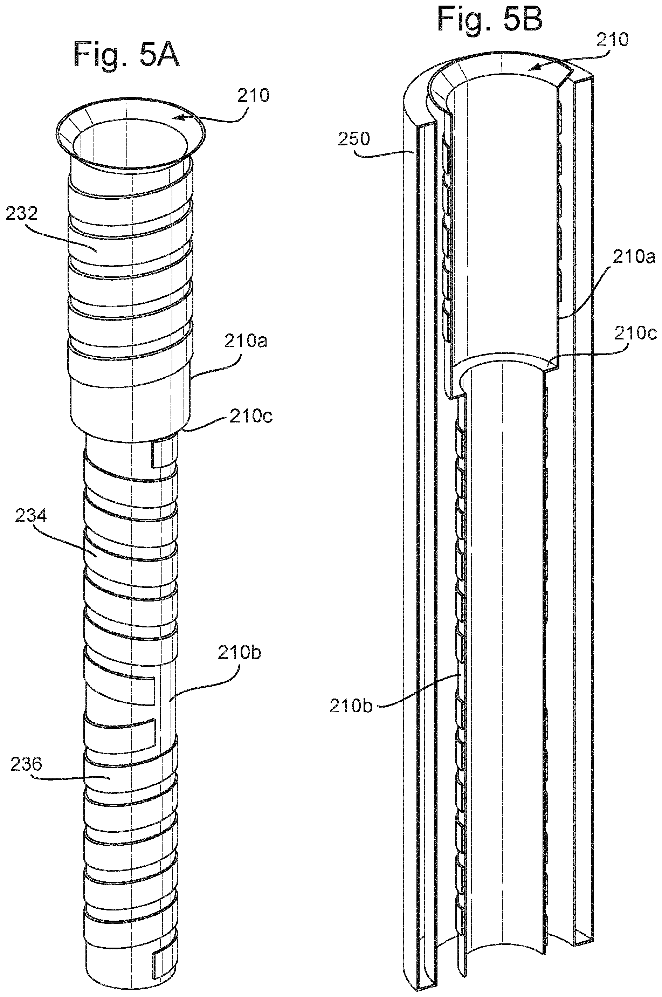

Figure 5A shows the heating tube 210 and coils 232, 234, 236 in a perspective view. As can be seen in Figure 5A, the first section 210a of the heating tube 210 in this example comprises a portion which tapers to the first diameter from a larger diameter at the proximal end of the heating tube 210. Figure 5B shows the heating tube 210, coils 232, 234, 236 and the isolation tube 250 in a perspective cross-sectional view.

Although in this example the device 200 is a device which uses resistive heating to heat the heating tube 210 and thereby heat the consumables 120a, 120b, in another example, the device 200 may use inductive heating. When inductive heating is used, inductive elements may be located exterior to the isolation tube 250. In one example using inductive heating, a number of coils, e.g. three coils corresponding with the respective sections of the heating tube 210 with which the coils 232, 234, 236 correspond, may be located exterior to the isolation tube 250, for example being wound around an exterior of the isolation tube 250. The heating tube 210 may formed of a metal such as stainless steel or aluminium.

The respective diameters of the first section 210a and the second section 210b of the heating tube may be such that when the first consumable 120a is received by the heating tube 210, the first section 210a is in close proximity with the first consumable 120a. Similarly, when the second consumable 120b is received by the heating tube 210, the second section 210b may be in close proximity with the second consumable 120b. The heating tube 210 may be referred to as a stepped heating tube in some examples. This allows whichever of the consumables 120a, 120b which is inserted into the heating chamber 210 to be effectively heated by a respective portion of the heating tube. The user may operate the device according to which of the consumables 120a, 120b is to be used. For example, the user may interact with the operating switch 112 to select a use mode suitable for the inserted consumable. Alternatively, or additionally, the device 200 may detect which of the consumables 120a, 120b is inserted in the heating tube 210 and may operate in an appropriate mode accordingly.

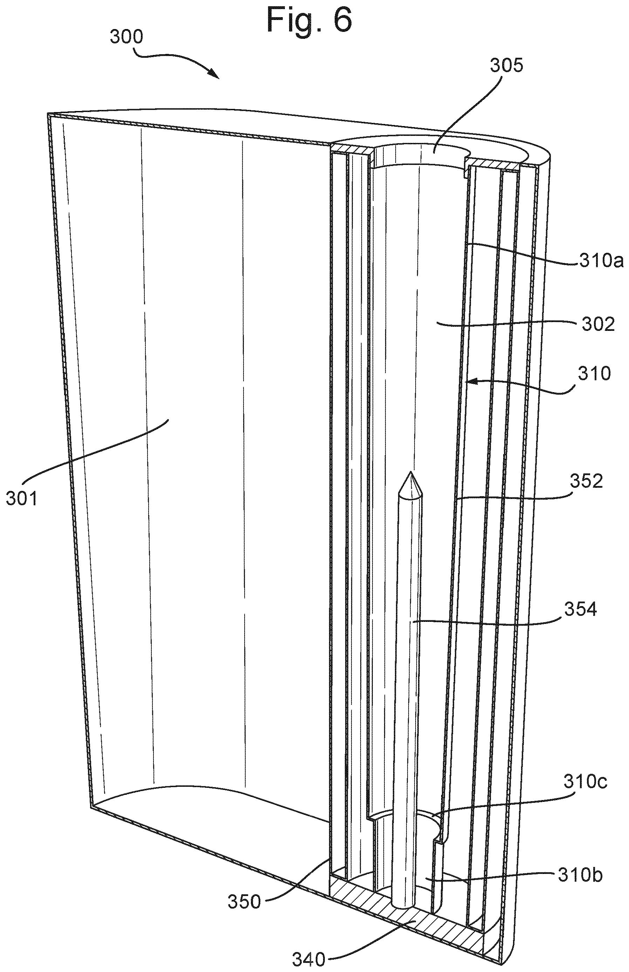

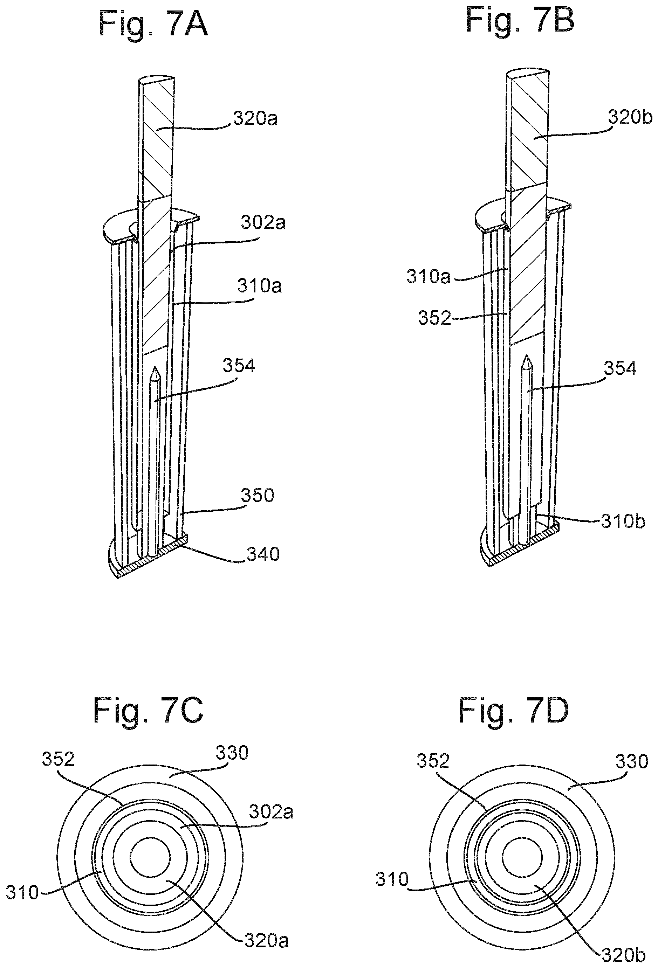

Figure 6 shows a third device 300 which is an example according to the invention. The third device 300 may comprise any of the features described for the first device 100 and a description of these features will not be repeated. Figure 7A and Figure 7B show in a perspective view, in cross-section, a portion of the third device 300 comprising a heating tube 310 for receiving differently-dimensioned consumables. Figure 7A shows a first consumable 320a received in the heating chamber 310 while Figure 7B shows a second consumable 320b received in the heating chamber 310. Other components of the device 300 are omitted from Figure 7A and Figure 7B for clarity. Figures 7C and 7D show, respectively, schematic top-down views of the arrangements shown in Figure 7A and 7B.

The heating tube of the third device 300 is a stepped heating tube 310 which is similar to the heating tube 210 of the second device 200. The heating tube 310 comprises a first section 310a at its proximal end and a second section 310b at its distal end. In the same manner as the heating tube 210 of the second device 200, the second section 310b has a diameter less than that of the first section 310a. Accordingly, a step 310c is formed between the first section 310a and the second section 310b.

The heating tube 310 of the third device 300 is configured to receive the first consumable 320a and the second consumable 320b. In the same manner as described for the second device 200, the first consumable 320a has a smaller diameter than the second consumable 320b and has a length such that it can be inserted into the second section 310b of the heating tube 310 where it may in some examples abut a distal component 340. The second consumable 320b has a larger diameter than the first consumable 320a which is such that the second consumable 320b cannot be inserted into the second section 310b. Accordingly, a distal end of the second consumable 320b is configured to abut the step 310c.

**In** this example, the second section 310b of the heating tube 310 is much shorter in length than the first section 310a. **In** some examples, the second section 310b may function as a stepped arrangement for the consumables 320a, 320b but may not provide any heating to the consumables. For example, the first section 310a of the heating tube may heat the consumables 320a, 320b while the second section 320b does not. The first consumable 320a and the second consumable 320b have different lengths. **In** some examples, the difference in length between the first consumable 320a and the second consumable 320b may be substantially equal to the length of the second section 310b. This may allow for the respective proximal ends of the first consumable 320a and the second consumable 320b to protrude from the opening 305 to the heating tube 302 by substantially equal amounts. For example, in some examples, a filter portion and various other components of the consumables 320a, 320b may be arranged at the same distance from the respective proximal ends of the consumables 320a, 320b. Thus, having the consumables protrude from the opening 305 by substantially equal amounts may allow ventilation holes and the like to be positioned in the same axial location with respect to the opening 305.

The third device 300 is configured to heat the heating tube 310 by use of a heating foil 352 which is located on the exterior of the heating tube 310. The heating foil 352 is a resistive heater. Further, a heating element 354 is located in the heating chamber 302. The heating element 354 protrudes from the distal component 340 into the heating chamber 302. The heating element 354 is configured to enter whichever of the first consumable 320a and the second consumable 320b is received in the heating tube 310. The heating element 354 is a resistive heating element and may be operated to heat the aerosol-generating material from within the respective consumable. The heating element 354 may be controllable independently of the heating foil 352.

In this example, the second consumable 320b has a diameter such that when inserted in the chamber 302 it is in close contact with the interior wall of the first section 310a of the heating tube 310. This may allow the second consumable 320b to be effectively heated by heating tube 310. However, since the first consumable 320a has a diameter which is less than that of the second consumable 320b, a gap 302a is formed between the first consumable 320a and the interior wall of the first section 310a. Therefore, heat transfer from the heating tube 310 may not be as effective for the first consumable 320a as for the second consumable 310b. The heating element 354 may, in some examples, allow for more even heating of the first consumable 320a and the second consumable 320b than if heating were done by the heating tube 310 alone. For example, the heating element 354 may be operated to heat the aerosol-generating material when the first consumable 320a is received in the chamber 302 but not when the second consumable 320b is received in the chamber 302.

Figure 7C illustrates the gap 302a which is formed between the first consumable 320a and the interior wall of the first section 310a of when the first consumable 320a is received in the heating tube 310. Figure 7D shows the second consumable 320b being received in the heating tube 310 and being in close proximity with the interior wall of the first section 310a.

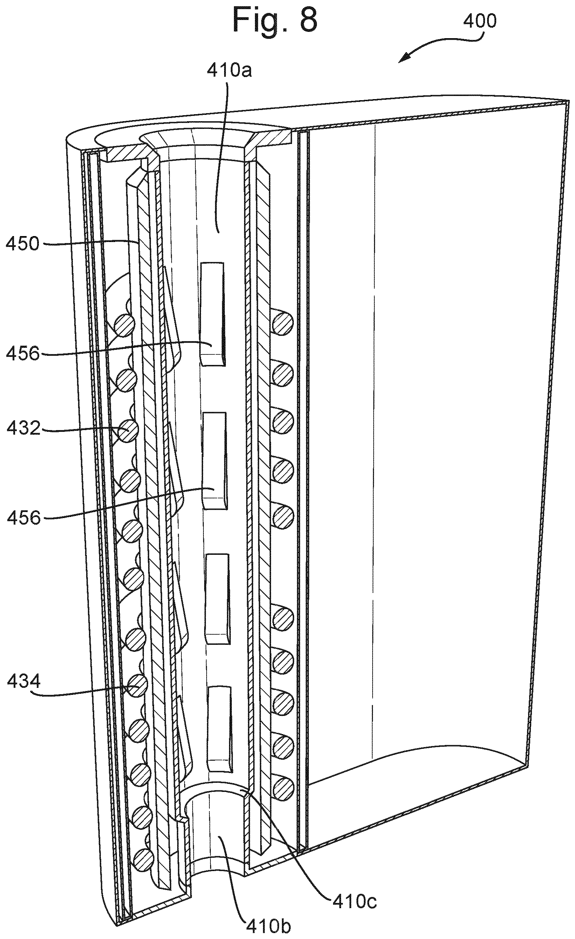

Figure 8 shows a fourth device 400 according to one example of the invention. The fourth device 400 shares certain features described with reference to earlier described example devices and description of these features will not be repeated. Certain components of the fourth device 400 have been omitted from Figure 8 for the purpose of clarity.

The fourth device 400 is an inductive heating device comprising a heating tube 410. A first coil 432 and second coil 434 surround the heating tube 410 and are configured to heat, respectively, a proximal portion and a distal portion of the heating tube 410. The heating tube 410 comprises a set of flexible members 456 which are configured to engage whichever of the first consumable 120a or the second consumable 120b is received in the heating tube 410. In this example, there are four rows of flexible members though it will be appreciated that in other examples there may be any other number of flexible members 456. The flexible members 456 are conductive and are configured to conduct heat from the heating tube 410 to whichever consumable is received in the heating tube 410. The flexible members 456 protrude radially inward and are configured to flex when either of the first consumable 120a or second consumable 120b is inserted into the heating tube 410. This may provide for effective heat transfer from the inductively heated heating tube 410, i.e. the susceptor tube, and the received consumable. Since the flexible members 456 are configured to engage both the larger diameter second consumable 120b and the smaller diameter first consumable 120a, the first and second consumables 120a, 120b may be more equally heated than if relying on conduction from the heating tube 410 alone. That is, as described in previous examples, the second consumable 120b may be in closer proximity with the interior wall of the heating tube 410 than the first consumable 120a. The flexible members 456 may therefore provide for more even heating of both consumables 120a, 120b.

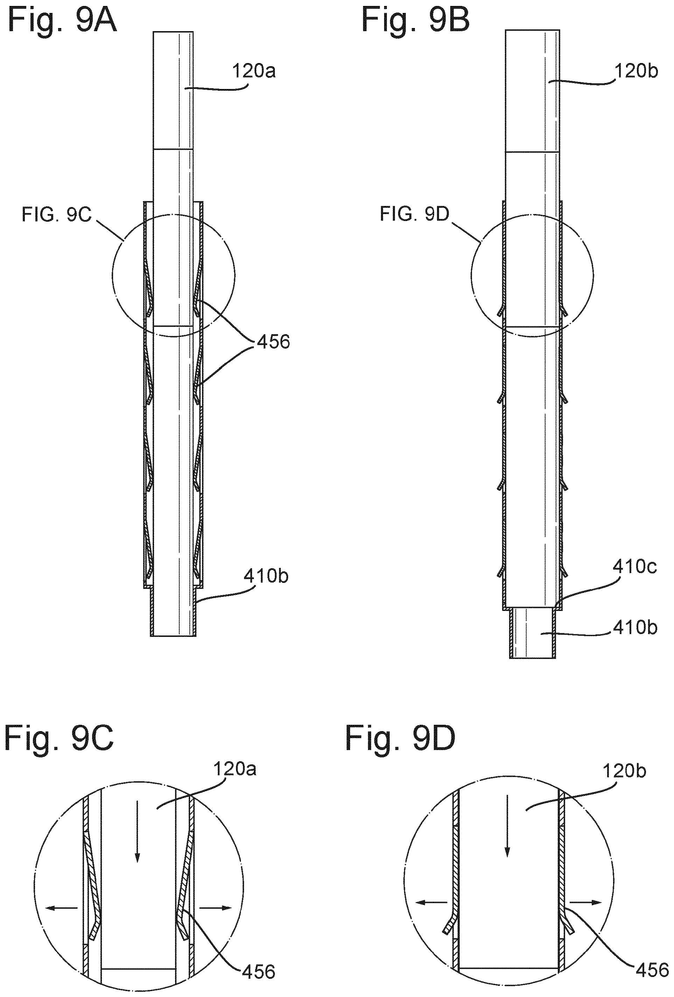

Figure 9A shows the first consumable 120a received in the heating tube 410 while Figure 9C shows a close-up cutaway view of the flexible members 456 engaging the first consumable 120a. Similarly, Figure 9B shows the second consumable 120b received in the heating tube 410 and Figure 9D shows a close-up cutaway view of the flexible members 456 engaging the first consumable 120a. It can be seen from Figures 9A to 9D that the flexible members 456 protrude further radially inwardly to engage the first consumable 120a than to engage the second consumable 120b.

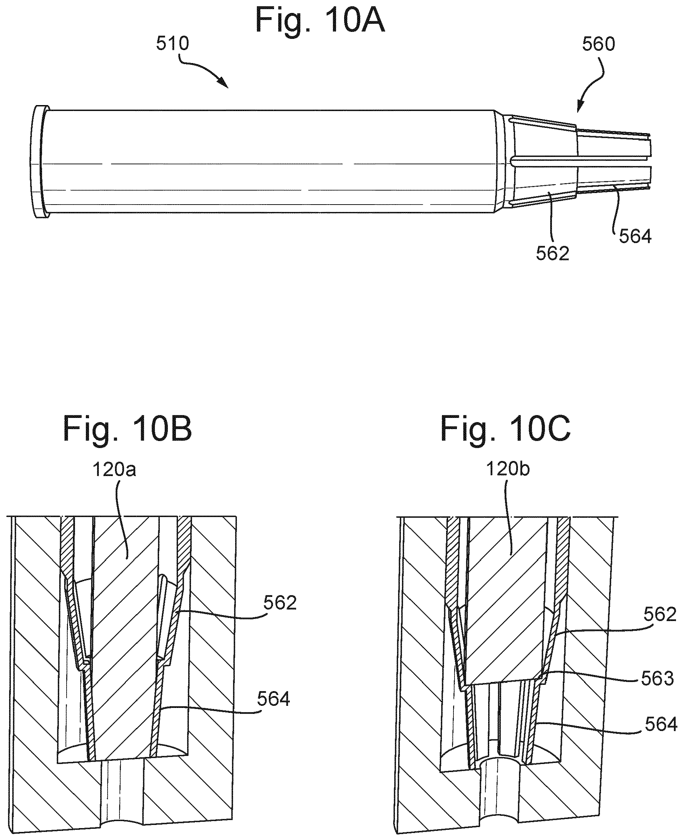

Figures 10A to 10C show a heating tube 510 according to another example of the invention. A second section 560 of the heating tube 510 is configured to grip a distal end of whichever of the first and second consumables 120a, 120b is received in the heating tube 510. The second section 560 is flexible and is configured to be biased to grip the distal end of an inserted consumable. The second section 560 comprises a proximal section 562 for receiving and gripping a distal end of the second consumable 120b and a distal section 564 for receiving and gripping a distal end of the first consumable 120a. A step 563 separates the distal section 564 from the proximal section 562 and prevents the second consumable 120b being inserted into the distal section 564 while allowing the first consumable 120a to be inserted into the distal section 564. The second section 560 comprises a plurality of slits and is slightly tapered and is configured to flex to fit to the diameter of an inserted consumable and to grip the distal end of the consumable. The heating tube 510 may be a susceptor tube or may act as a heating tube for a device using resistive heating.

The above embodiments are to be understood as illustrative examples of the invention. Further embodiments of the invention are envisaged. It is to be understood that any feature described in relation to any one embodiment may be used alone, or in combination with other features described, and may also be used in combination with one or more features of any other of the embodiments, or any combination of any other of the embodiments.

## Claims

1. A non-combustible aerosol provision device (200) for generating an aerosol from an aerosol-generating material, the non-combustible aerosol provision device (200) comprising:
a chamber (102) for receiving a consumable (120a, 120b) comprising aerosol-generating material to enable the non-combustible aerosol provision device (200) to generate an aerosol from the aerosol-generating material;
wherein the chamber (102) is configured to accommodate, one at a time, a first consumable (120a) comprising aerosol-generating material at a first axial position in the chamber (102) and a second consumable (120b) comprising aerosol-generating material having different dimensions to those of the first consumable (120a) at a second axial position in the chamber (102) wherein the chamber (102) is configured to accommodate the first consumable (120a) having a first width at the first axial position in the chamber (102) and the second consumable (120b) having a second width at the second axial position in the chamber (102), wherein the first width is less than the second width, wherein the chamber (102) comprises a first section (210a) and a second section (210b), wherein the second section (210b) has a width smaller than a width of the first section (210a), wherein the first section (210a) and the second section (210b) are tubular sections arranged end to end to form the chamber (102), wherein the device (200) comprises an opening (105) at a proximal end of the first section (210a) configured to allow the insertion of the first consumable (120a) and the second consumable (120b) into at least the first section (210a), and
wherein the proximal end of the second section (210b) defines a stop (210c) to be abutted by a distal end of the second consumable (120b) to prevent the second consumable (120b) entering the second section (210b) and thereby to position the second consumable (120b) at the second axial position in the chamber (102).

2. The non-combustible aerosol provision device (200) according to claim 1, wherein the device (200) comprises a first heating arrangement (232) arranged to heat the first section (210a) to generate the aerosol and a second heating arrangement (234) arranged to heat the second section (210b) to generate the aerosol.

3. The non-combustible aerosol provision device (200) according to claim 2, wherein the first heating arrangement (232) and the second heating (234) arrangement are controllable independently of one another to heat, respectively, the first section (210a) and the second section (210b).

4. The non-combustible aerosol provision device (200) according to claim 1 or claim 2, comprising a first distal stop (240) for positioning a distal end of the first consumable (120a) and a second distal stop (210c) for positioning a distal end of the second consumable (120b), the first distal stop (240) and the second distal stop (210c) being located at different axial positions in the chamber (102).

5. The non-combustible aerosol provision device (200) according to claim 4, wherein the second distal stop (210c) comprises a step surrounding a recess extending distally of the step, and wherein the first distal stop (240) is located in the recess.

6. The non-combustible aerosol provision device (200) according to claim 5, wherein the recess comprises a gripping arrangement which is biased to grip the distal end of the first consumable (120a) when the distal end of the first consumable (120a) is accommodated in the recess.

7. The non-combustible aerosol provision device (200) according to any of claim 1 to claim 6, wherein the chamber (102) is defined by a heating tube (210) configured to be heated and to transfer heat to a consumable (120a, 120b) comprising aerosol-generating material received in the chamber (102) to thereby heat the aerosol generating material and generate the aerosol.

8. The non-combustible aerosol provision device (200) according to claim 7, wherein the heating tube (210) is a susceptor element configured to be inductively heated by an inductive element of the device (200) to thereby heat the aerosol-generating material received in the chamber (102), or wherein the heating tube (210) is configured to be resistively heated to thereby heat the aerosol-generating material received in the chamber (102).

9. The non-combustible aerosol provision device (200) according to claim 7 or claim 8, wherein the device (200) comprises one or more heat transfer elements (456) for transferring heat from the heating tube (210) to the consumable (120a, 120b), wherein the one or more heat transfer elements (456) are configured to protrude radially inward of the interior walls of the heating tube (210) to engage consumables (120a, 120b) having different diameters.

10. The non-combustible aerosol provision device (200) according to claim 9, wherein the one or more heat transfer elements (456) are flexible elements configured to flex to engage consumables (120a, 120b) having different diameters.

11. The non-combustible aerosol provision device (200) according to any of claim 1 to claim 10, wherein the device (200) comprises a heating arrangement (354) configured to be inserted into the consumable (120a, 120b) to heat the aerosol-generating material in the consumable (120a, 120b) to thereby generate the aerosol from the aerosol-generating material.

12. The non-combustible aerosol provision device (200) according to any of claim 1 to claim 11 wherein the device (200) is a tobacco heating product and wherein the first consumable (120a) and the second consumable (120b) each are consumables comprising tobacco to be heated by the device (200) to generate the aerosol.

13. A non-combustible aerosol provision system comprising a non-combustible aerosol provision device (200) according to any of claim 1 to claim 12 and at least one of the first consumable (120a) containing aerosol-generating material, and the second consumable (120b) containing aerosol-generating material, wherein the first consumable (120a) and the second consumable (120b) are configured to be received, one at a time, by the non-combustible aerosol provision device (200) to allow the device (200) to generate aerosol from the aerosol-generating material.

## Patentansprüche

1. Vorrichtung zur Bereitstellung eines Aerosols ohne Verbrennung (200) zum Erzeugen eines Aerosols aus einem aerosolerzeugenden Material, die Vorrichtung zur Bereitstellung eines Aerosols ohne Verbrennung (200) umfassend:
eine Kammer (102) zum Aufnehmen eines Verbrauchsmaterials (120a, 120b), umfassend aerosolerzeugendes Material, um der Vorrichtung zur Bereitstellung eines Aerosols ohne Verbrennung (200) zu ermöglichen, aus dem aerosolerzeugenden Material ein Aerosol zu erzeugen;
wobei die Kammer (102) dazu konfiguriert ist, eines nach dem anderen, ein erstes Verbrauchsmaterial (120a), umfassend aerosolerzeugendes Material, an einer ersten axialen Position in der Kammer (102) und ein zweites Verbrauchsmaterial (120b), umfassend aerosolerzeugendes Material, das unterschiedliche Abmessungen zu denen des ersten Verbrauchsmaterials (120a) aufweist, an einer zweiten axialen Position in der Kammer (102) aufzunehmen,
wobei die Kammer (102) dazu konfiguriert ist, das erste Verbrauchsmaterial (120a), das an der ersten axialen Position in der Kammer (102) eine erste Breite aufweist, und das zweite Verbrauchsmaterial (120b), das an der zweiten axialen Position in der Kammer (102) eine zweite Breite aufweist, aufzunehmen,
wobei die erste Breite geringer als die zweite Breite ist, wobei die Kammer (102) einen ersten Abschnitt (210a) und einen zweiten Abschnitt (210b) umfasst, wobei der zweite Abschnitt (210b) eine Breite aufweist, die kleiner als eine Breite des ersten Abschnitts (210a) ist, wobei der erste Abschnitt (210a) und der zweite Abschnitt (210b) rohrförmige Abschnitte sind, die Ende an Ende angeordnet sind, um die Kammer (102) zu bilden,
wobei die Vorrichtung (200) eine Öffnung (105) an einem proximalen Ende des ersten Abschnitts (210a) umfasst, die dazu konfiguriert ist, ein Einführen des ersten Verbrauchsmaterials (120a) und des zweiten Verbrauchsmaterials (120b) in mindestens den ersten Abschnitt (210a) zu ermöglichen, und
wobei das proximale Ende des zweiten Abschnitts (210b) einen Anschlag (210c) definiert, an dem ein distales Ende des zweiten Verbrauchsmaterials (120b) anstößt, um zu verhindern, dass das zweite Verbrauchsmaterial (120b) in den zweiten Abschnitt (210b) eindringt, und um dadurch das zweite Verbrauchsmaterial (120b) an der zweiten axialen Position in der Kammer (102) zu positionieren.

2. Vorrichtung zur Bereitstellung eines Aerosols ohne Verbrennung (200) nach Anspruch 1, wobei die Vorrichtung (200) eine erste Heizanordnung (232), die angeordnet ist, um den ersten Abschnitt (210a) zu erwärmen, um das Aerosol zu erzeugen, und eine zweite Heizanordnung (234), die angeordnet ist, um den zweiten Abschnitt (210b) zu erwärmen, um das Aerosol zu erzeugen, umfasst.

3. Vorrichtung zur Bereitstellung eines Aerosols ohne Verbrennung (200) nach Anspruch 2, wobei die erste Heizanordnung (232) und die zweite Heizanordnung (234) unabhängig voneinander steuerbar sind, um den ersten Abschnitt (210a) bzw. den zweiten Abschnitt (210b) zu erwärmen.

4. Vorrichtung zur Bereitstellung eines Aerosols ohne Verbrennung (200) nach Anspruch 1 oder Anspruch 2, umfassend einen ersten distalen Anschlag (240) zum Positionieren eines distalen Endes des ersten Verbrauchsmaterials (120a) und einen zweiten distalen Anschlag (210c) zum Positionieren eines distalen Endes des zweiten Verbrauchsmaterials (120b), wobei sich der erste distale Anschlag (240) und der zweite distale Anschlag (210c) an unterschiedlichen axialen Positionen in der Kammer (102) befinden.

5. Vorrichtung zur Bereitstellung eines Aerosols ohne Verbrennung (200) nach Anspruch 4, wobei der zweite distale Anschlag (210c) eine Stufe umfasst, die eine Aussparung umgibt, die sich distal von der Stufe erstreckt, und wobei sich der erste distale Anschlag (240) in der Aussparung befindet.

6. Vorrichtung zur Bereitstellung eines Aerosols ohne Verbrennung (200) nach Anspruch 5, wobei die Aussparung eine Greifanordnung umfasst, die vorgespannt ist, um das distale Ende des ersten Verbrauchsmaterials (120a) zu ergreifen, wenn das distale Ende des ersten Verbrauchsmaterials (120a) in der Aussparung aufgenommen ist.

7. Vorrichtung zur Bereitstellung eines Aerosols ohne Verbrennung (200) nach einem von Anspruch 1 bis Anspruch 6, wobei die Kammer (102) durch ein Heizrohr (210) definiert ist, das dazu konfiguriert ist, erwärmt zu werden und Wärme auf ein Verbrauchsmaterial (120a, 120b), umfassend aerosolerzeugendes Material, zu übertragen, das in der Kammer (102) aufgenommen ist, um dadurch das aerosolerzeugende Material zu erwärmen und das Aerosol zu erzeugen.

8. Vorrichtung zur Bereitstellung eines Aerosols ohne Verbrennung (200) nach Anspruch 7, wobei das Heizrohr (210) ein Suszeptorelement ist, das dazu konfiguriert ist, durch ein induktives Element der Vorrichtung (200) induktiv erwärmt zu werden, um dadurch das in der Kammer (102) aufgenommene aerosolerzeugende Material zu erwärmen, oder wobei das Heizrohr (210) dazu konfiguriert ist, durch einen Widerstand erwärmt zu werden, um dadurch das in der Kammer (102) aufgenommene aerosolerzeugende Material zu erwärmen.

9. Vorrichtung zur Bereitstellung eines Aerosols ohne Verbrennung (200) nach Anspruch 7 oder Anspruch 8, wobei die Vorrichtung (200) ein oder mehrere Wärmeübertragungselemente (456) zum Übertragen von Wärme von dem Heizrohr (210) auf das Verbrauchsmaterial (120a, 120b) umfasst, wobei das eine oder die mehreren Wärmeübertragungselemente (456) dazu konfiguriert sind, von den Innenwänden des Heizrohrs (210) radial nach innen zu ragen, um mit Verbrauchsmaterialien (120a, 120b), die unterschiedliche Durchmesser aufweisen, in Eingriff zu kommen.

10. Vorrichtung zur Bereitstellung eines Aerosols ohne Verbrennung (200) nach Anspruch 9, wobei das eine oder die mehreren Wärmeübertragungselemente (456) flexible Elemente sind, die dazu konfiguriert sind, sich zu biegen, um mit Verbrauchsmaterialien (120a, 120b), die unterschiedliche Durchmesser aufweisen, in Eingriff zu kommen.

11. Vorrichtung zur Bereitstellung eines Aerosols ohne Verbrennung (200) nach einem von Anspruch 1 bis Anspruch 10, wobei die Vorrichtung (200) eine Heizanordnung (354) umfasst, die dazu konfiguriert ist, in das Verbrauchsmaterial (120a, 120b) eingeführt zu werden, um das aerosolerzeugende Material in dem Verbrauchsmaterial (120a, 120b) zu erwärmen, um dadurch aus dem aerosolerzeugenden Material das Aerosol zu erzeugen.

12. Vorrichtung zur Bereitstellung eines Aerosols ohne Verbrennung (200) nach einem von Anspruch 1 bis Anspruch 11, wobei die Vorrichtung (200) ein Tabakerwärmungsprodukt ist und wobei das erste Verbrauchsmaterial (120a) und das zweite Verbrauchsmaterial (120b) jeweils Tabak umfassende Verbrauchsmaterialien sind, die von der Vorrichtung (200) erwärmt werden sollen, um das Aerosol zu erzeugen.

13. System zur Bereitstellung eines Aerosols ohne Verbrennung, umfassend eine Vorrichtung zur Bereitstellung eines Aerosols ohne Verbrennung (200) nach einem von Anspruch 1 bis Anspruch 12 und mindestens eines von dem ersten Verbrauchsmaterial (120a), das aerosolerzeugendes Material enthält, und dem zweiten Verbrauchsmaterial (120b), das aerosolerzeugendes Material enthält, wobei das erste Verbrauchsmaterial (120a) und das zweite Verbrauchsmaterial (120b) dazu konfiguriert sind, von der Vorrichtung zur Bereitstellung eines Aerosols ohne Verbrennung (200) aufgenommen zu werden, um der Vorrichtung (200) zu ermöglichen, aus dem aerosolerzeugenden Material Aerosol zu erzeugen.

## Revendications

1. Dispositif (200) de fourniture d'aérosol sans combustion pour générer un aérosol à partir d'un matériau générateur d'aérosol, le dispositif (200) de fourniture d'aérosol sans combustion comprenant :
une chambre (102) pour recevoir un consommable (120a, 120b) comprenant un matériau générateur d'aérosol pour permettre au dispositif (200) de fourniture d'aérosol sans combustion de générer un aérosol à partir du matériau générateur d'aérosol ;
dans lequel la chambre (102) est configurée pour accueillir, un à la fois, un premier consommable (120a) comprenant un matériau générateur d'aérosol à une première position axiale dans la chambre (102) et un deuxième consommable (120b) comprenant un matériau générateur d'aérosol ayant des dimensions différentes de celles du premier consommable (120a) à une deuxième position axiale dans la chambre (102)
dans lequel la chambre (102) est configurée pour accueillir le premier consommable (120a) ayant une première largeur à la première position axiale dans la chambre (102) et le deuxième consommable (120b) ayant une deuxième largeur à la deuxième position axiale dans la chambre (102),
dans lequel la première largeur est inférieure à la deuxième largeur, dans lequel la chambre (102) comprend une première section (210a) et une deuxième section (210b), dans lequel la deuxième section (210b) a une largeur inférieure à une largeur de la première section (210a), dans lequel la première section (210a) et la deuxième section (210b) sont des sections tubulaires agencées bout à bout pour former la chambre (102),
dans lequel le dispositif (200) comprend une ouverture (105) à une extrémité proximale de la première section (210a) configurée pour permettre l'insertion du premier consommable (120a) et du deuxième consommable (120b) dans au moins la première section (210a), et
dans lequel l'extrémité proximale de la deuxième section (210b) définit une butée (210c) destinée à être en butée avec une extrémité distale du deuxième consommable (120b) pour empêcher le deuxième consommable (120b) de pénétrer dans la deuxième section (210b) et ainsi pour positionner le deuxième consommable (120b) à la deuxième position axiale dans la chambre (102).

2. Dispositif (200) de fourniture d'aérosol sans combustion selon la revendication 1, dans lequel le dispositif (200) comprend un premier agencement de chauffage (232) agencé pour chauffer la première section (210a) pour générer l'aérosol et un deuxième agencement de chauffage (234) agencé pour chauffer la deuxième section (210b) pour générer l'aérosol.

3. Dispositif (200) de fourniture d'aérosol sans combustion selon la revendication 2, dans lequel le premier agencement de chauffage (232) et le deuxième agencement de chauffage (234) peuvent être commandés indépendamment l'un de l'autre pour chauffer, respectivement, la première section (210a) et la deuxième section (210b).

4. Dispositif (200) de fourniture d'aérosol sans combustion selon la revendication 1 ou la revendication 2, comprenant une première butée distale (240) pour positionner une extrémité distale du premier consommable (120a) et une deuxième butée distale (210c) pour positionner une extrémité distale du deuxième consommable (120b), la première butée distale (240) et la deuxième butée distale (210c) étant situées à différentes positions axiales dans la chambre (102).

5. Dispositif (200) de fourniture d'aérosol sans combustion selon la revendication 4, dans lequel la deuxième butée distale (210c) comprend un gradin entourant un évidement s'étendant distalement du gradin, et dans lequel la première butée distale (240) est située dans l'évidement.

6. Dispositif (200) de fourniture d'aérosol sans combustion selon la revendication 5, dans lequel l'évidement comprend un arrangement de préhension qui est sollicité pour saisir l'extrémité distale du premier consommable (120a) lorsque l'extrémité distale du premier consommable (120a) est accueillie dans l'évidement.

7. Dispositif (200) de fourniture d'aérosol sans combustion selon l'une quelconque de la revendication 1 à la revendication 6, dans lequel la chambre (102) est définie par un tube chauffant (210) configuré pour être chauffé et pour transférer de la chaleur à un consommable (120a, 120b) comprenant un matériau générateur d'aérosol reçu dans la chambre (102) afin de chauffer ainsi le matériau générateur d'aérosol et générer l'aérosol.

8. Dispositif (200) de fourniture d'aérosol sans combustion selon la revendication 7, dans lequel le tube chauffant (210) est un élément suscepteur configuré pour être chauffé par induction par un élément inductif du dispositif (200) afin de chauffer ainsi le matériau générateur d'aérosol reçu dans la chambre (102), ou dans lequel le tube chauffant (210) est configuré pour être chauffé par résistance afin de chauffer ainsi le matériau générateur d'aérosol reçu dans la chambre (102).

9. Dispositif (200) de fourniture d'aérosol sans combustion selon la revendication 7 ou la revendication 8, dans lequel le dispositif (200) comprend un ou plusieurs éléments de transfert de chaleur (456) pour transférer la chaleur du tube chauffant (210) au consommable (120a, 120b), dans lequel les un ou plusieurs éléments de transfert de chaleur (456) sont configurés pour faire saillie radialement vers l'intérieur des parois intérieures du tube chauffant (210) pour être en prise avec des consommables (120a, 120b) ayant des diamètres différents.

10. Dispositif (200) de fourniture d'aérosol sans combustion selon la revendication 9, dans lequel les un ou plusieurs éléments de transfert de chaleur (456) sont des éléments flexibles configurés pour fléchir afin d'être en prise avec des consommables (120a, 120b) ayant des diamètres différents.

11. Dispositif (200) de fourniture d'aérosol sans combustion selon l'une quelconque de la revendication 1 à la revendication 10, dans lequel le dispositif (200) comprend un agencement de chauffage (354) configuré pour être inséré dans le consommable (120a, 120b) pour chauffer le matériau générateur d'aérosol dans le consommable (120a, 120b) pour ainsi générer l'aérosol à partir du matériau générateur d'aérosol.

12. Dispositif (200) de fourniture d'aérosol sans combustion selon l'une quelconque de la revendication 1 à la revendication 11 dans lequel le dispositif (200) est un produit chauffant de tabac et dans lequel le premier consommable (120a) et le deuxième consommable (120b) sont chacun des consommables comprenant du tabac destiné à être chauffé par le dispositif (200) pour générer l'aérosol.

13. Système de fourniture d'aérosol sans combustion comprenant un dispositif (200) de fourniture d'aérosol sans combustion selon l'une quelconque de la revendication 1 à la revendication 12 et au moins l'un du premier consommable (120a) contenant un matériau générateur d'aérosol et du deuxième consommable (120b) contenant un matériau générateur d'aérosol, dans lequel le premier consommable (120a) et le deuxième consommable (120b) sont configurés pour être reçus, un à la fois, par le dispositif (200) de fourniture d'aérosol sans combustion pour permettre au dispositif (200) de générer un aérosol à partir du matériau générateur d'aérosol.
